# EUROPEAN PATENT SPECIFICATION

(11) **EP 2 927 328 B1**
(45) Date of publication and mention of the grant of the patent: **10.10.2018**
(21) Application number: 14305477.3
(22) Date of filing: 01.04.2014
(51) Int. Cl.: C12Q 1/6886

(54) **SSEA4 and ST3GAL2 as chemotherapeutic drug response biomarkers**
SSEA4 und ST3GAL2 als Reaktions-Biomarker chemotherapeutischer Medikamente
SSEA4 et ST3GAL2 comme biomarqueurs de réponse à un médicament chimiothérapeutique

(43) Date of publication of application: 07.10.2015
(73) Proprietor: Miltenyi Biotec GmbH, 51429 Bergisch Gladbach (DE); XenTech, 91000 Evry (FR)
(72) Inventor: Hardt, Olaf, 51061 Köln (DE); Bosio, Andeas, 50933 Köln (DE); Aloia, Andrea, 50676 Köln (DE); Judde, Jean-Gabriel, 94110 Arcueil (FR); Petrova, Evgeniya, 75003 Paris (FR); Cairo, Stefano, 91310 Longpont sur Orge (FR); Deas, Olivier, 91400 Gometz la Ville (FR)
(74) Representative: Biervert, Christian

(56) References cited:
- EP-A1- 2 036 988
- WO-A1-2013/152301
- S. GOTTSCHLING ET AL: "Stage-specific embryonic antigen-4 is expressed in basaloid lung cancer and associated with poor prognosis", EUROPEAN RESPIRATORY JOURNAL, vol. 41, no. 3, 27 June 2012 (2012-06-27), pages 656-663, XP055142330, ISSN: 0903-1936, DOI: 10.1183/09031936.00225711
- H MA ET AL: "Modification of sialylation is associated with multidrug resistance in human acute myeloid leukemia", ONCOGENE, 17 February 2014 (2014-02-17), XP055142208, ISSN: 0950-9232, DOI: 10.1038/onc.2014.7
- ZHAO YONGFU; LI YANPING; MA HONGYE; DONG WEIJIE; ZHOU HUIMIN; SONGXIAOBO; ZHANG JIANING; JIA LI: "Modification of Sialylation Mediates the Invasive Properties andChemosensitivity of Human Hepatocellular Carcinoma", MOLECULAR & CELLULAR PROTEOMICS, vol. 13, no. 2, 1 February 2014 (2014-02-01), pages 520-563, XP008172269, ISSN: 1535-9476
- ANDREA ALOIA ET AL: "The sialyl-glycolipid stage-specific embryonic antigen 4 marks a subpopulation of chemotherapy-resistant breast cancer cells with mesenchymal features", BREAST CANCER RESEARCH, vol. 17, no. 1, 25 November 2015 (2015-11-25), XP055423969, DOI: 10.1186/s13058-015-0652-6

## Description

### Field of the invention

The present invention relates to methods for predicting the outcome for chemotherapeutic treatment of cancer in an individual and biomarkers for the same.

### Background

In the field of oncology, biomarkers are used to calculate the prognosis of a malignancy, predict suitable treatments, monitor disease progression, and evaluate treatment impact. The range of used biomarkers is extremely wide. Morphological or histological characteristics but also molecular markers on the protein and nucleic acid level (DNA/RNA) are frequently used.

Whereas good markers for the majority of targeted therapies exist, for most tumor entities there is a lack of markers that can be used to predict the response for systemic therapies like chemotherapy. In breast cancer, chemotherapy is predominately used for stage 2-4 disease, being particularly beneficial in estrogen receptor-negative (ER-) disease. Usually, several drugs are given in combinations, and one of the most common treatments is cyclophosphamide plus doxorubicin (Adriamycin®), known as A/C. In general, chemotherapy works by unspecifically destroying fast-growing/fast-replicating cells. In the case of A/C cell death is induced by causing DNA damage during replication. Due to the general mechanism of action for the majority of chemotherapy agents, these drugs next to targeting tumor cells also damage fast-growing normal cells where they cause serious side effects like hair loss or liver and gastrointestinal complications.
WO2013/152301A1 discloses the use of O-glycan biosynthesis pathway (OGBP) genes including ST3GAL2 for determining the prognosis of a patient with ovarian cancer, wherein a favorable prognosis comprises an increased likelihood of survival after treatment with primary chemotherapy.
EP2036988A1 discloses biomarkers for risk of developing recurrent gynecologic cancer towards a chemotherapeutic agent. The genes disclosed are AKR1C1, MLPH, ESR1, PGR, COMP, DCN, IGKC, CCL5, FBN1 and/or UBE2C.
Gottschling et al. (2012, European Respiratory Journal 41:656-663) disclose high SSEA4 levels in lung cancer with relapse and describe the surface antigen as a marker for bad prognosis.

Although less than 25% of breast cancer patients benefit from chemotherapeutic treatment (CLIFFORD A. HUDIS and LUCA GIANNI, The Oncologist 2011;16(suppl 1):1-11), this systemic approach is still used as standard care. Because of the severe side effects, it would be highly beneficial to identify markers which can be used to predict the response to chemotherapy preventing treatment of de-novo resistant tumors.

### Summary of the invention

Surprisingly, it was found that SSEA4 (stage-specific embryonic antigen-4), a sialyl-glycolipid, and ST3GAL2, the synthesizing enzyme of SSEA4, are diagnostic and prognostic drug response biomarkers in cancer.

Expression of SSEA4 and/or ST3GAL2 on/in cancerous cells predicts the outcome for chemotherapeutic treatment of these cells. SSEA4 and/or ST3GAL2 can be used as biomarkers in a method to predict resistance to chemotherapy in an individual with cancer wherein said cancer is selected from the group consisting of human breast cancer, human renal cell carcinoma (RCC) and human ovarian cancer if the biomarker is SSEA4 and wherein said cancer is selected from the group consisting of human breast cancer and if the biomarker is ST3GAL2 and wherein the chemotherapeutic drugs used in said chemotherapeutic treatment are DNA synthesis and transcription inhibitors.

### Brief description of the drawings

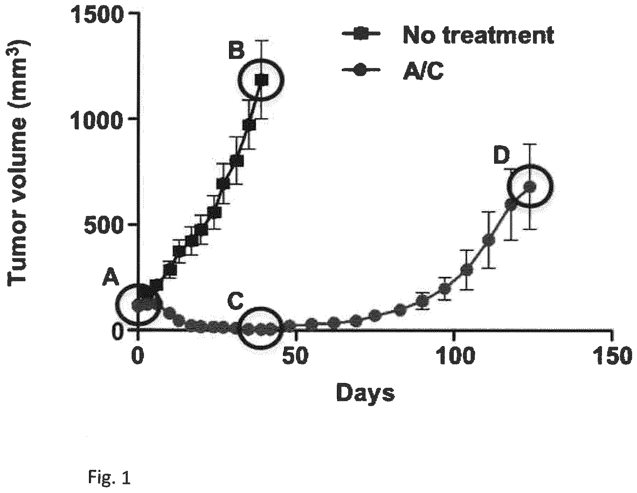

### Fig. 1: Study design for chemotherapy treatment of xenograft tumors

Upon stable engraftment of the human tumors (pre-treatment stage, A) mice were treated using a standard care doxorubicin /cyclophosphamide (A/C) combination. After tumor shrinking, the residual nodules were removed (residual tumor stage, C). At the same day, tumors of untreated mice were removed (untreated stage, B) serving as direct controls. In addition, a group of animals with residual tumors were kept until the disease relapsed (regrown stage, D).

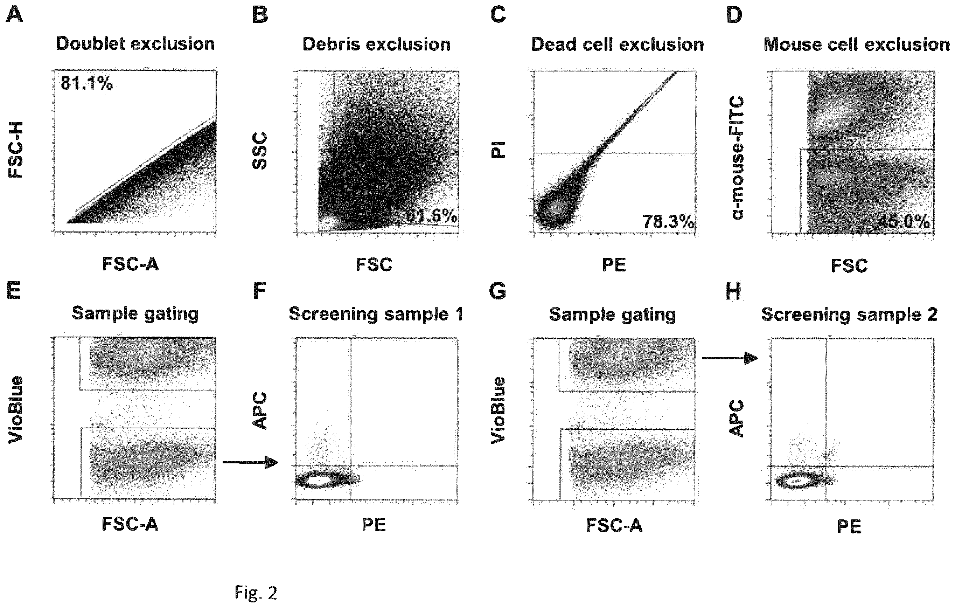

### Fig. 2: Representative gating strategy for flow cytometry-based marker analysis of dissociated xenograft tumor tissue

Tumor tissue was dissociated to obtain a single cell suspension while preserving cell surface epitopes. The sample was stained for mouse specific markers to exclude cells of murine origin from the analysis as well as for the screening candidates and analyzed by multi-parameter flow cytometry. Doublets were excluded by FSC-A/FSC-H gating (A), debris was excluded by FSC/SSC gating (B), dead cells were excluded by gating off PI⁺ events (C), and mouse cells were excluded by gating on α-mouse-FITC-negative events (D). When screening two samples in parallel, one of the samples was labeled using a UV-dye allowing for subsequent separation of the events of each sample by gating on the VioBlue-channel fluorescence intensity (E-H).

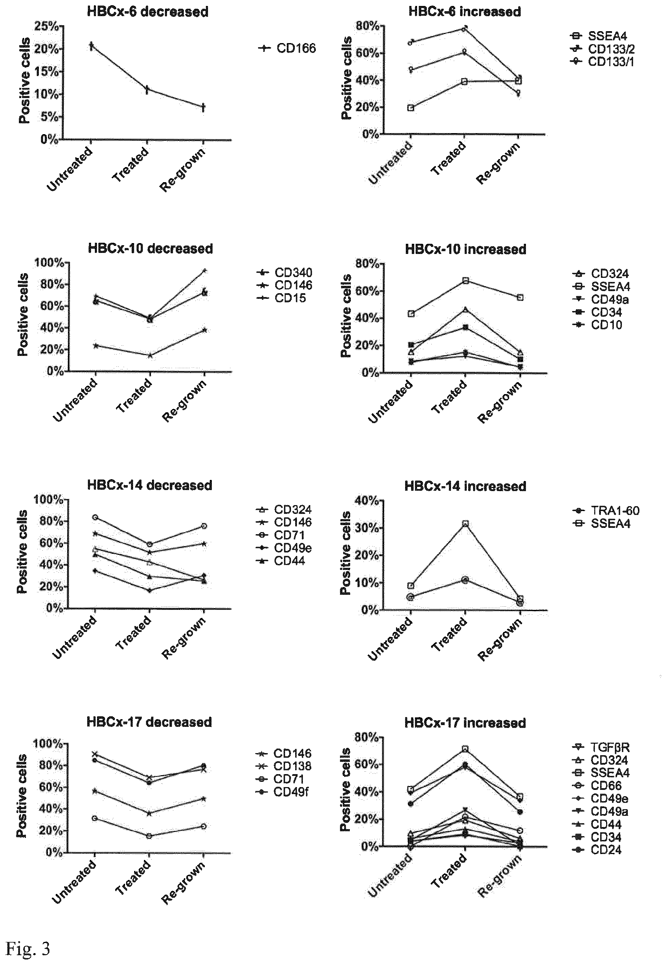

### Fig. 3: Identification of differentially regulated cell surface markers during chemotherapeutic treatment

The expression patterns of cell surface markers in xenograft tumors from four independent breast cancer patients, HBCx-6 (A, B), HBCx-10 (C, D), HBCx-14 (E, F), and HBCx-17 (G, H), were analyzed by multi-parameter flow cytometry at three time points, the residual tumor stage, the untreated stage, and the re-grown stage. In total, the expression of 13 markers (2 to 9 per tumor model) was increased (B, D, F, H) and the expression of 10 markers (1 to 5 per tumor model) was decreased (A, C, E, G) during chemotherapy.

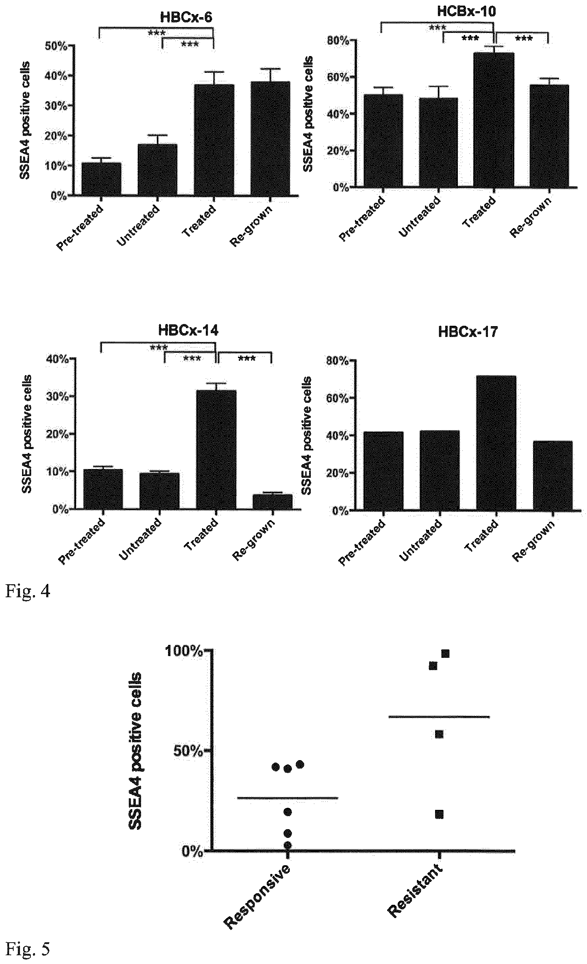

### Fig. 4: Expression of SSEA4 during chemotherapeutic treatment of xenograft tumors

The expression of SSEA4 during chemotherapeutic treatment of xenograft tumors from four independent breast cancer patients, HBCx-6, HBCx-10, HBCx-14, and HBCx-17, was analyzed by multi-parameter flow cytometry at four time points, the pre-treatment stage, the residual tumor cell stage and the parallel untreated stage, and the re-growth stage. The number of SSEA4 positive cells was significantly (p < 0.001, n = 8) enriched upon A/C treatment in all analyzed tumors. In addition, in three out of four tumor models, HBCx-10, HBCx-14, and HBCx-17, the fraction of positive cells was reduced to background levels when tumors relapsed.
***p< 0.001

### Fig. 5: Expression of SSEA4 in tumors sensitive or resistant for chemotherapeutic treatment

Tumors which are sensitive (n = 6) or resistant (n = 4) to A/C treatment were analyzed for expression of SSEA4. Three out of the four resistant tumor models showed higher percentages of SSEA4 positive cells than all of the six sensitive tumors. In two of the resistant tumor models, almost all of the cells expressed SSEA4.

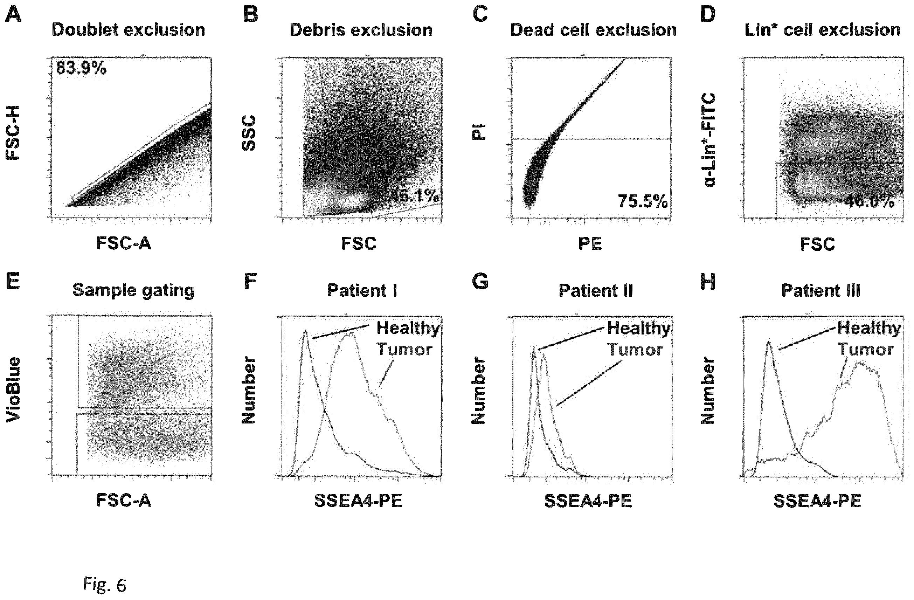

### Fig. 6: Expression of SSEA4 in RCC and healthy kidney tissue

Clear cell renal cell carcinoma (RCC), shown to be de novo resistant to chemotherapy in more than 95% of patients, was investigated for expression of SSEA4. Primary RCC as well as healthy kidney tissue from the same patient was dissociated and analyzed by multi-parameter flow cytometry. Doublets were excluded by FSC-A/FSC-H gating (A), debris was excluded by FSC/SSC gating (B), dead cells were excluded by gating off PI⁺ events (C), and lineage positive cells were excluded by gating on α-Lin-FITC negative events (D). In each patient, healthy and tumor tissue was analyzed in parallel. Therefore, one of the samples was labeled using a UV-dye allowing for subsequent separation of the events of each sample by gating on the VioBlue-channel fluorescence intensity (E-H). In all of the analyzed patients (n = 3) the expression of SSEA4 was strongly increased in the tumor tissue with almost all tumor cells expressing this marker (F-H).
*α-Lin-FITC = CD45-FITC, CD31-FITC, CD235a (Glycophorin A)-FITC

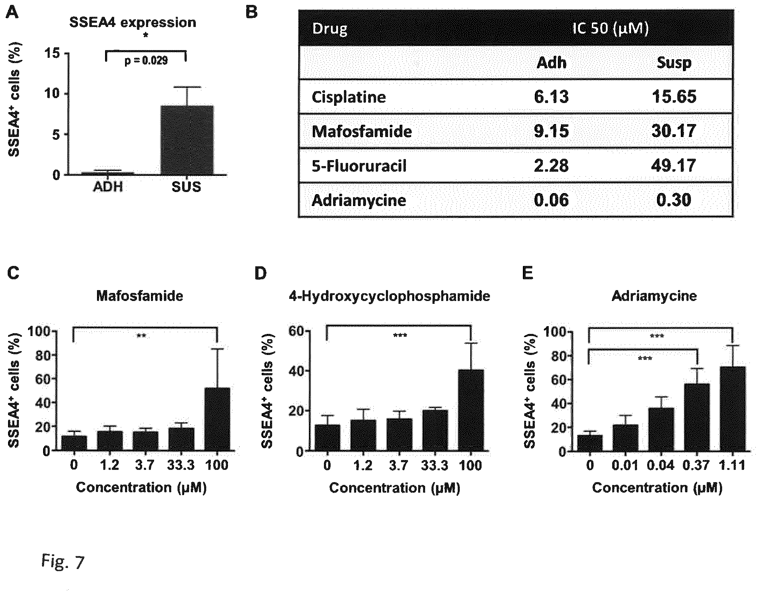

### Fig. 7: Expression of SSEA4 during chemotherapeutic treatment of breast cancer cells in vitro

Cell lines containing different amounts of SSEA4 positive cells were treated with chemotherapeutic drugs in vitro. A primary tumor cell line derived from model HBCx-17 showed reproducible growth partly in an adherent and in a suspension phenotype. The cells growing in suspension showed a significantly (p = 0.029) higher SSEA4 expression compared to the adherent cells (A). To evaluate the sensitivity of both subpopulations to chemotherapeutic treatment, the IC50 values (n = 3) for commonly used drugs were measured. For Cisplatine, Mafosfamide, 5-Fluorouracil, and Adriamycine®, the suspension cells showed higher IC50 values, indicating an increased resistance to those drugs (B). To directly examine the phenotype of cells surviving the treatment, another purely adherent cell line derived from model HBCx-17 was treated with increasing concentrations of either Mafosfamide (C), 4-Hydroxycyclophosphamide (D), or Adriamycine® (E) (n = 4). In every case, the surviving population showed a significantly higher fraction of SSEA4 positive cells.
** p < 0.01, ***p< 0.001

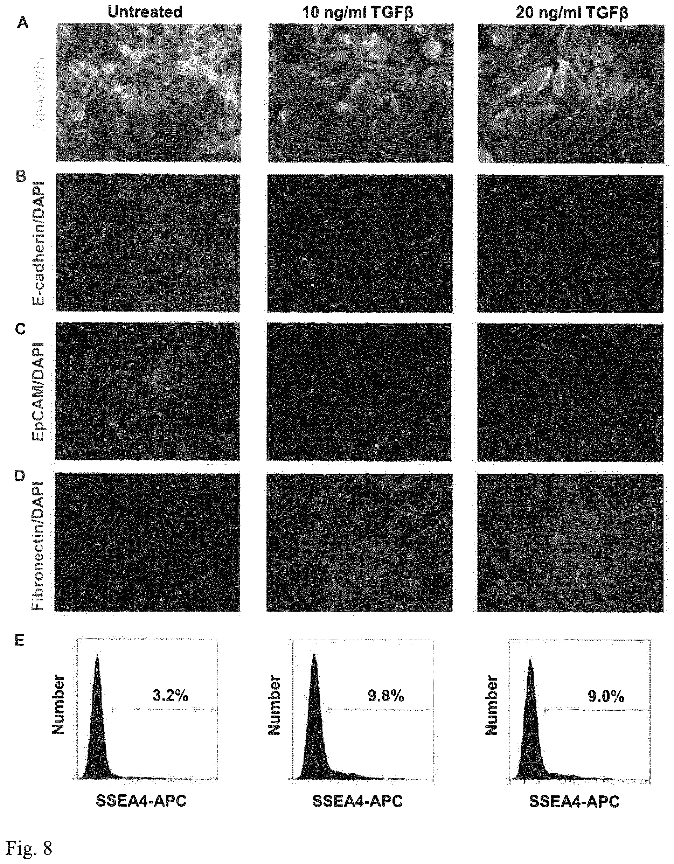

### Fig. 8: Expression of SSEA4 indicates a more mesenchymal phenotype

The expression of SSEA4 was evaluated upon EMT induction. To induce a mesenchymal transition, the epithelial breast cell line MCF10A was treated with TGFβ1 at concentrations of 10 and 20 ng/ml. Upon treatment, almost all cells changed their morphology from a compacted colony type to an elongated fibroblastic shape (A). F-actin was stained by Phalloidin to visualize the cytoskeleton architecture. The epithelial markers E-cadherin (B) and EpCAM (C) were downregulated whereas the mesenchymal marker Fibronectin (D) was upregulated upon treatment. The fraction of SSEA4 positive cells was increased upon EMT induction (E) verifying that expression of SSEA4 indicates a more mesenchymal phenotype.

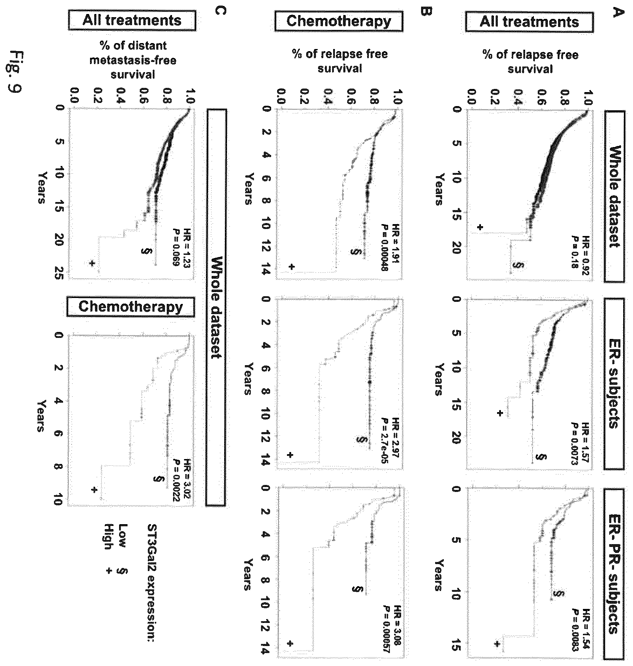

### Fig. 9: Expression of ST3GAL2 correlates with prognosis for clinical outcome of breast cancer patients

Due to the nature of the SSEA4 epitope, its expression cannot be directly monitored by transcriptome profiling. Therefore, the prognostic value of its synthetizing enzyme ST3GAL2 was evaluated using a large public clinical microarray database of breast tumors from 2,977 patients (Gyorffy B et al., Breast Cancer Res Treatment, 2010 Oct;123(3):725-31.). When using the whole dataset, no difference in the clinical outcome was observed whereas highly significant differences (p < 0.01) towards a poorer prognosis for patients expressing high levels of ST3GAL2 in the ER- patients, PR- patients, and double negative patients were found independent of the treatment (A). When focusing on patients treated with chemotherapy, a highly significant reduction of relapse-free survival independent of the tumor subtype (p < 0.01, HR 1.91), in ER- patients (p < 0.01, HR 2.97), and in double negative patients (p < 0.01, HR 3.08) was observed in patients expressing high levels of ST3GAL2(B). Also, when applying distant metastasis-free survival as primary endpoint, patients treated with chemotherapy had a worse prognosis when expressing high levels of ST3GAL2 (C).

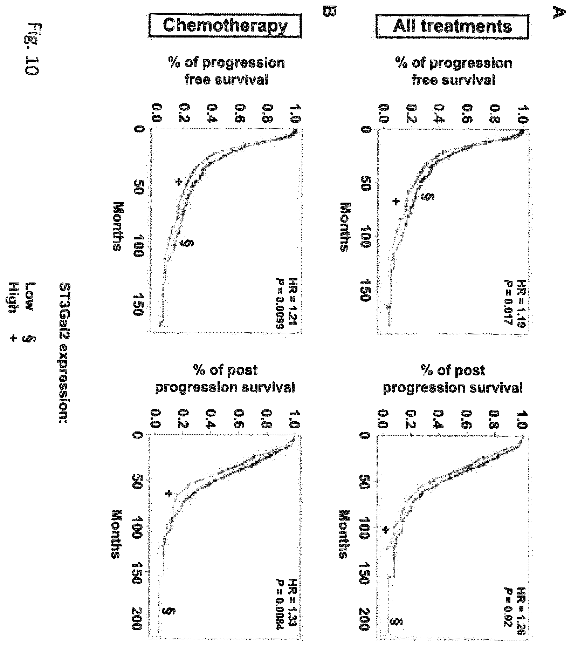

### Fig. 10: Expression of ST3GAL2 correlates with prognosis for clinical outcome of ovarian cancer patients

To evaluate the prognostic value of ST3GAL2 expression in an independent tumor entity, a public clinical microarray database of ovarian tumors from 1,464 patients was analyzed (Gyorffy B et al., Endocrine-Related Cancer. 2012 Apr 10;19(2):197-208.). When using the whole dataset, a significant difference towards a worse clinical outcome was observed with respect to progression free survival (p < 0.05) as well as post progression survival (p < 0.05, A). When focusing on patients treated with chemotherapy, the level of significance was further increased for both endpoints (p < 0.01, Figure B).

### Detailed description of the invention

Unexpectedly, we identified that SSEA4 and ST3GAL2, the synthesizing enzyme of SSEA4, are biomarkers for chemotherapy resistance to cancer in an individual. The expression of SSEA4 and/or ST3GAL2 on/in cancerous cells can be used for prediction or estimation of the response to chemotherapeutic treatment of said cells in an individual wherein said cancer is selected from the group consisting of human breast cancer, human renal cell carcinoma (RCC) and human ovarian cancer if the biomarker is SSEA4 and wherein said cancer is selected from the group consisting of human breast cancer if the biomarker is ST3GAL2 and wherein the chemotherapeutic drugs used in said chemotherapeutic treatment are DNA synthesis and transcription inhibitors.

Therefore, in a first aspect, the invention provides the use of SSEA4 and/or ST3GAL2 as biomarkers for assessing the outcome for chemotherapeutic treatment of a cancer in an individual wherein said cancer is selected from the group consisting of human breast cancer, human renal cell carcinoma (RCC) and human ovarian cancer if the biomarker is SSEA4 and wherein said cancer is selected from the group consisting of human breast cancer if the biomarker is ST3GAL2 and wherein the chemotherapeutic drugs used in said chemotherapeutic treatment are DNA synthesis and transcription inhibitors.
The assessment may be of diagnostic value.
In another aspect, the invention provides a method for assessing the prognosis associated to resistance to chemotherapy in an individual with cancer, the method comprising the steps of
a) Providing a sample to be tested
b) Detecting expression of SSEA4 in said sample
Wherein the expression of SSEA4 in said sample is indicative of a poor prognosis and wherein said cancer is selected from the group consisting of human breast cancer, human renal cell carcinoma (RCC) and human ovarian cancer, and wherein the chemotherapeutic drugs used in said chemotherapy are DNA synthesis and transcription inhibitors.

Preferentially, the expression of SSEA4 on more than 50% of the cancerous cells of said sample to be tested is indicative of poor prognosis.

Understandably, the method of the present disclosure allows the prognosis associated to sensitivity to chemotherapy (good prognosis) in an individual with cancer wherein said cancer is selected from the group consisting of human breast cancer, human renal cell carcinoma (RCC) and human ovarian cancer if the biomarker is SSEA4 and wherein the chemotherapeutic drugs used in said chemotherapeutic treatment are DNA synthesis and transcription inhibitors.

This is the case if only very few cells, preferentially no cells of the sample to be tested have the biomarker SSEA4.

The presence or expression of SSEA4 on the cancerous cells may be determined by any method known in the art suitable for determining the presence or detecting the expression of a biomarker on cells such as staining with an antibody or fragment thereof. Preferentially the antibody is a monoclonal antibody. Methods for staining cells with antibodies or fragments thereof are well known in the art such as flow cytometry, immunohistochemistry or immunocytochemistry.

In another aspect, the invention provides a method for assessing the prognosis associated to resistance to chemotherapy in an individual with cancer, the method comprising the steps of
a) Providing a sample to be tested
b) Detecting the expression level of ST3GAL2 in said sample
c) Comparing the detected expression level to a control level
d) Determining said prognosis based on the comparison of c)
Wherein the control level is a good prognosis control level and an increase of the expression level compared to the control level is determined as a poor prognosis, and wherein said cancer is selected from the group consisting of human breast cancer and human ovarian cancer, and wherein the chemotherapeutic drugs used in said chemotherapy are DNA synthesis and transcription inhibitors.
Preferentially, an increase of the expression level of at least 50% compared to the control level is determined as indicator of poor prognosis.
The expression of the ST3GAL2 is determined on mRNA level or protein level.

Understandably, an increase of the expression level of less than 50% compared to the control level is determined as indicator of good prognosis. The method of the present disclosure allows the prognosis associated to sensitivity to chemotherapy (good prognosis) in an individual having a cancer, wherein said cancer is selected from the group consisting of human breast cancer and human ovarian cancer, and wherein the chemotherapeutic drugs used in said chemotherapy are DNA synthesis and transcription inhibitors.
This is the case if no increase of the expression level of ST3GAL2 between the cells of the sample to be tested and the control level is detectable.

In another aspect, the invention provides a method for assessing the prognosis associated to resistance to chemotherapy in an individual with cancer, the method comprising the steps of
a) Providing a sample to be tested
b) Detecting the activity level of the ST3GAL2 protein in said sample
c) Comparing the detected activity level to a control level
d) Determining said prognosis based on the comparison of c)
Wherein the control level is a good prognosis control level and an increase of the activity level compared to the control level is determined as a poor prognosis, and wherein said cancer is selected from the group consisting of human breast cancer and human ovarian cancer, and wherein the chemotherapeutic drugs used in said chemotherapy are DNA synthesis and transcription inhibitors.

Preferentially, an increase of the activity level of at least 50% compared to the control level is determined as indicator of poor prognosis.
The activity of the ST3GAL2 protein is determined on the protein level.

Understandably, an increase of the activity level of less than 50% compared to the control level is determined as indicator of good prognosis. The method of the present disclosure allows the prognosis associated to sensitivity to chemotherapy (good prognosis) in an individual with cancer, wherein said cancer is selected from the group consisting of human breast cancer and human ovarian cancer, and wherein the chemotherapeutic drugs used in said chemotherapy are DNA synthesis and transcription inhibitors.
This is the case if no increase of the activity level of ST3GAL2 between the cells of the sample to be tested and the control level is detectable.

The chemotherapeutic treatment comprises the application of chemotherapeutical drugs which are DNA synthesis and transcription inhibitors. More preferentially, said chemotherapeutic treatment comprises the application of chemotherapeutical drugs selected from the group consisting of Cisplatine, Mafosfamide, Fluorouracil/5-FU, Doxorubicin, and Docetaxel. Most preferentially, said chemotherapeutic treatment comprises the application of the chemotherapeutical drug combination doxorubicin /cyclophosphamide (A/C).

SSEA4 and/or ST3GAL2 are significant prognostic biomarkers that indicate resistance of cancerous cells to chemotherapy in an individual, wherein said cancer is selected from the group consisting of human breast cancer, human renal cell carcinoma (RCC) and human ovarian cancer if the biomarker is SSEA4 and wherein said cancer is selected from the group consisting of human breast cancer and human ovarian cancer if the biomarker is ST3GAL2 and wherein the chemotherapeutic drugs used in said chemotherapeutic treatment are DNA synthesis and transcription inhibitors.
Preferentially, SSEA4 and/or ST3GAL2 are significant prognostic biomarkers associated to resistance to chemotherapy in an individual having human breast cancer. More preferentially, SSEA4 and/or ST3GAL2 are significant prognostic biomarkers that indicate resistance to chemotherapy in an individual having human breast cancer of the subtype "triple negative breast cancer".

Expression of the gene ST3GAL2 can be determined at the nucleic acid level, using methods known in the art such as Northern blot hybridization analysis, reverse-transcription-based PCR assays, RNA microarrays, or DNA microarrays. Expression can also be determined at the protein level, i.e., by measuring the level of the polypeptide encoded by the gene ST3GAL2, or the biological activity thereof (the synthesis of SSEA4). Such methods are well known in the art and include, but are not limited to, e.g., immunoassays such as enzyme-linked immunosorbent assays (ELISAs), Western blot, immunohistochemistry, immunocytochemistry, substrate based turnover assays, or protein microarrays.
Since SSEA4 is a sialyl-glycolipid the presence of SSEA4 cannot be directly measured on nucleic acid level.

### Definitions

Unless defined otherwise, technical and scientific terms used herein have the same meaning as commonly understood by one of ordinary skill in the art to which this invention belongs.

Herein, the term "prognosis" refers to a forecast as to the probable outcome associated to the success of a chemotherapeutic treatment of a cancer in an individual. Accordingly, a less favorable, negative, poor prognosis is defined by a resistance of the cancerous cells to the chemotherapeutic treatment resulting in a lower post-treatment survival term or survival rate of the individual. Conversely, a positive, favorable, or good prognosis is defined by sensitivity of the cancerous cells to the chemotherapeutic treatment resulting in an elevated post-treatment survival term or survival rate. Therefore a "poor prognosis" predictor is associated with increased risk that an individual suffering from a cancer is or will be resistant to chemotherapy applied for treatment of said cancer. Therefore "good prognosis" predicts a good probability that a patient suffering from a cancer is or will be sensitive to chemotherapy applied for treatment of said cancer.
The terms "assessing the prognosis" or "assessing the outcome" refer to the ability of predicting, forecasting or correlating a given detection or measurement with a future outcome associated with response to chemotherapeutic treatment of the cancer of the patient.
The present method for assessing prognosis associated to resistance to chemotherapy of a cancer in an individual is - among others - intended to be used clinically in making decisions concerning treatment modalities, including chemotherapeutic intervention, and diagnostic criteria such as disease staging.
The term "resistance to chemotherapy" means that some of the cancerous cells of the sample to be tested (at least one cell) is resisting the intended effect of the chemotherapeutic treatment.
The term "sensitivity to chemotherapeutic treatment" means that the cancerous cells of the sample to be tested are sensitive to the intended effect of the chemotherapeutic treatment.
The sample used for the present method is a biological sample, preferentially it is an individual-derived sample comprising tumor tissue or cancerous cells. The sample may be obtained from an individual (patient) at various time points, including before, during, and/or after chemotherapeutic treatment. Preferentially, the sample to be tested in the present method is obtained before chemotherapeutical treatment. Preferentially, the sample to be tested comprises a single cell suspension comprising at least 1%, at least 10%, at least 20%, at least 30%, at least 40%, at least 50%, at least 60%, at least 70%, at least 80%, at least 90, at least 95%, at least 99% cancerous cells. Tumor tissue for the sample to be tested may be generated invasively from an individual suffering from cancer and the tumor tissue may be dissociated into cell suspension using methods known in the art. E.g. tumor tissue is dissociated with human Tumor Dissociation Kit in combination with the gentleMACS Dissociator (both Miltenyi Biotec GmbH). After dissociation, the cells are resuspended in buffer for flow cytometry. Other methods for generation of the sample are available in the art and further examples are given below. As an alternative to flow cytometric analysis, immunohistochemistry or immunocytochemistry can be used for detection of SSEA4 and/or ST3GAL2 expression with or without prior tissue dissociation.
Preferentially, these cells are viable. But, these cells can also be fixed cells which may be used for subsequent nucleic acids or protein extraction. The invention is illustrated mainly isolating human tumor tissue which has been implanted into mice. However, it encompasses isolation of tumor tissue in general, e.g. directly from human patients suffering from a cancer.

Any methods available in the art for "detecting expression", "detecting the expression level", or "detecting the activity level" of biomarkers are encompassed herein. The expression of a biomarker of the invention can be detected on a nucleic acid level or a protein level (both for ST3GAL2) or a glycolipid level (SSEA4). By "detecting expression", "detecting expression level" or "detecting the activity level" is intended determining the quantity or presence of a biomarker. Thus, "detecting expression", "detecting the expression level" or "detecting the activity level" encompasses instances where a biomarker is determined not to be expressed, not to be detectably expressed, expressed at a low level, expressed at a normal level, or overexpressed. In order to determine overexpression, the sample to be tested may be compared with a corresponding sample that originates from a healthy person or healthy tissue of the same patient. That is, the "normal" level of expression is the level of expression of the biomarker in, for example, a breast tissue sample from an individual not afflicted with breast cancer or region not infiltrated by the tumor tissue (control level). Such a sample can be present in standardized form. In some embodiments, determination of biomarker (over-) expression requires no comparison between the sample to be tested and a corresponding sample (control level). For example, detection of expression of a biomarker such as SSEA4 indicative of a prognosis for resistance to chemotherapeutic treatment of a cancer in an individual may preclude the need for comparison to a corresponding sample (control level) that e.g. originates from a healthy person or healthy tissue of a patient. Methods for "detecting expression", "detecting the expression level", or "detecting the activity level" of the biomarkers of the invention comprise any methods that determine the quantity or the presence of the biomarkers either at the nucleic acid level or protein level or at glycolipid level. Such methods are well known in the art and include but are not limited to western blots, northern blots, southern blots, ELISA, immunoprecipitation, immunofluorescence, flow cytometry, immunohistochemistry, nucleic acid hybridization techniques, nucleic acid reverse transcription methods, and nucleic acid amplification methods. Methods for detecting the activity level of proteins are also well known in the art, such as ELISA or substrate based turnover assays.
A normal "control level" is an expression profile of biomarkers such as the ST3GAL2 gene typically found in a cell population from an individual known not to be suffering from a cancer or healthy tissue of a patient. An increase in the level of expression of the biomarker such as ST3GAL2 gene in a sample to be tested from an individual suffering from a cancer (patient) in comparison to expression from a normal control sample indicates an elevated probability of resistance to chemotherapy (poor prognosis) for the cancer.
For example, increase in expression levels of ST3GAL2 of at least 10%, at least 25%, at least 50%, at least 75%, at least 100% or more indicates the poor prognosis.
Difference between the expression levels of the sample to be tested from a patient and the control level can be normalized to the expression level of control nucleic acids, e.g., housekeeping genes, whose expression levels are known not to differ depending on the cancerous or non-cancerous state of the cell. Exemplary control genes include, but are not limited to, beta-actin, glyceraldehyde 3 phosphate dehydrogenase, and ribosomal protein P1.

For the method of assessing the prognosis associated to resistance or sensitivity to chemotherapy in an individual having a cancer using the biomarker SSEA4, in general, the presence of SSEA4 on the cancerous cells in the sample to be tested indicates the resistance of these cells to chemotherapy. At a threshold of 50% or more cells among the fraction of cancerous cells in the sample bearing the SSEA4 on the cell surface, the prediction of resistance against chemotherapy for the specific malignancy under investigation is extremely poor (see example 2).

The term "tumor" is known medically as a neoplasm. Not all tumors are cancerous; benign tumors do not invade neighboring tissues and do not spread throughout the body.

The term "cancer" is known medically as a malignant neoplasm. Cancer is a broad group of diseases involving unregulated cell growth. In cancer, cells divide and grow uncontrollably, forming malignant tumors, and invading nearby parts of the body. The cancer may also spread to more distant parts of the body through the lymphatic system or bloodstream. There are over 200 different known cancers that affect humans.

The terms "Chemotherapy" or "chemotherapeutic treatment" refer to the treatment of cancer (cancerous cells) with one or more cytotoxic anti-neoplastic drugs ("chemotherapeutic agents" or "chemotherapeutic drugs") as part of a standardized regimen. Chemotherapy may be given with a curative intent or it may aim to prolong life or to palliate symptoms. It is often used in conjunction with other cancer treatments, such as radiation therapy, surgery, and/or hyperthermia therapy. Traditional chemotherapeutic agents act by killing cells that divide rapidly, one of the main properties of most cancer cells. This means that chemotherapy also harms cells that divide rapidly under normal circumstances: cells in the bone marrow, digestive tract, and hair follicles. This results in the most common side-effects of chemotherapy: myelosuppression (decreased production of blood cells, hence also immunosuppression), mucositis (inflammation of the lining of the digestive tract), and alopecia (hair loss).

Some newer anticancer drugs (for example, various monoclonal antibodies) are not indiscriminately cytotoxic, but rather target proteins that are abnormally expressed in cancer cells and that are essential for their growth. Such treatments are often referred to as "targeted therapy" (as distinct from classic chemotherapy) and are often used alongside traditional chemotherapeutic agents in antineoplastic treatment regimens.

Types of classic chemotherapeutic drugs to which the terms "chemotherapeutic drugs" and "chemotherapy" as used herein refer are:
Alkylating agents: Alkylating agents are the oldest group of chemotherapeutics in use today. They are so named because of their ability to alkylate many molecules, including proteins, RNA and DNA. This ability to bind covalently to DNA or RNA via their alkyl group is the primary cause for their anti-cancer effects. This leads to a form of programmed cell death called apoptosis. Alkylating agents will work at any point in the cell cycle and thus are known as cell cycle-independent drugs. For this reason the effect on the cell is dose dependent; the fraction of cells that die is directly proportional to the dose of drug. The subtypes of alkylating agents are the nitrogen mustards, nitrosoureas, tetrazines, aziridines, cisplatins and derivatives, and non-classical alkylating agents. Nitrogen mustards include mechlorethamine, cyclophosphamide, melphalan, chlorambucil, ifosfamide and busulfan. Nitrosoureas include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide. Aziridines include thiotepa, mytomycin and diaziquone (AZQ). Cisplatin and derivatives include cisplatin, carboplatin and oxaliplatin. They impair cell function by forming covalent bonds with the amino, carboxyl, sulfhydryl, and phosphate groups in biologically important molecules. Non-classical alkylating agents include procarbazine and hexamethylmelamine. Mafosfamideis an oxazaphosphorine (cyclophosphamide-like) alkylating agent under investigation as a chemotherapeutic drug.
Anti-metabolites: The terms "anti-metabolites" and "DNA synthesis and transcription inhibitors" as used herein have an interchangeable meaning and define are a group of molecules that impede DNA and RNA synthesis. Many of them have a similar structure to the building blocks of DNA and RNA. Anti-metabolites resemble either nucleobases or nucleosides, but have altered chemical groups. These drugs exert their effect by either blocking the enzymes required for DNA synthesis or becoming incorporated into DNA or RNA. By inhibiting the enzymes involved in DNA synthesis, they prevent mitosis because the DNA cannot duplicate itself. Also, after misincorporation of the molecules into DNA, DNA damage can occur and programmed cell death (apoptosis) is induced. Unlike alkylating agents, anti-metabolites are cell cycle dependent. This means that they only work during a specific part of the cell cycle, in this case S-phase (the DNA synthesis phase). For this reason, at a certain dose, the effect plateaus and proportionally no more cell death occurs with increased doses. Subtypes of the anti-metabolites are the anti-folates, fluoropyrimidines, deoxynucleoside analogues and thiopurines. The anti-folates include methotrexate and pemetrexed. The fluoropyrimidines include fluorouracil and capecitabine. Fluorouracil is a nucleobase analogue that is metabolised in cells to form at least two active products; 5-fluourouridine monophosphate (FUMP) and 5-fluoro-2'-deoxyuridine 5'-phosphate (fdUMP). FUMP becomes incorporated into RNA and fdUMP inhibits the enzyme thymidylate synthase; both of which lead to cell death. Capecitabine is a prodrug of 5-fluorouracil that is broken down in cells to produce the active drug. The deoxynucleoside analogues include cytarabine, gemcitabine, decitabine, vidaza, fludarabine, nelarabine, cladribine, clofarabine and pentostatin. The thiopurines include thioguanine and mercaptopurine
Anti-microtubule agents: Anti-microtubule agents are plant-derived chemicals that block cell division by preventing microtubule function. Vinca alkaloids and taxanes are the two main groups of anti-microtubule agents. The vinca alkaloids prevent the formation of the microtubules, whereas the taxanes prevent the microtubule disassembly. By doing so, they prevent the cancer cells from completing mitosis. Following this, cell cycle arrest occurs, which induces programed cell death (apoptosis). Vinca alkaloids are derived from the Madagascar periwinkle, Catharanthusroseus. Taxanes are natural and semi-synthetic drugs. The first drug of their class, paclitaxel, was originally extracted from the Pacific Yew tree, Taxusbrevifolia. Now this drug and another in this class, docetaxel, are produced semi-synthetically from a chemical found in the bark of another Yew tree; Taxusbaccata. These drugs promote microtubule stability, preventing their disassembly. Docetaxel exerts its effect during S-phase.
Topoisomerase inhibitors: Topoisomerase inhibitors are drugs that affect the activity of two enzymes; topoisomerase I and topoisomerase II. When the DNA double stranded helix is unwound, during DNA replication or translation for example, the adjacent unopened DNA winds tighter (supercoils), like opening the middle of a twisted rope. The stress caused by this effect is in part aided by the topoisomerase enzymes. They produce single or double strand breaks into DNA, reducing the tension in the DNA strand. This allows the normal unwinding of DNA to occur during replication or translation. Inhibition of topoisomerase I or II interferes with both of these processes. Two topoisomerase I inhibitors, irinotecan and topotecan, are semi-synthetically derived from camptothecin, which is obtained from the Chinese ornamental tree Camptothecaacuminata. Drugs that target topoisomerase II can be divided into two groups. The topoisomerase II poisons cause increased levels enzymes bound to DNA. This prevents DNA replication and translation, causes DNA strand breaks, and leads to programmed cell death (apoptosis). These agents include etoposide, doxorubicin, mitoxantrone and teniposide. The second group, catalytic inhibitors, are drugs that block the activity of topoisomerase II, and therefore prevent DNA synthesis and translation because the DNA cannot unwind properly. This group includes novobiocin, merbarone, and aclarubicin. Cytotoxic antibiotics: The cytotoxic antibiotics are a varied group of drugs that have various mechanisms of action. The group includes the anthracyclines and other drugs including actinomycin, bleomycin, plicamycin and mitomycin. Doxorubicin and daunorubicin were the first two anthracyclines, and were obtained from the bacterium Streptomyces peucetius. Derivatives of these compounds include epirubicin and idarubicin. Other clinically used drugs in the anthracyline group are pirarubicin, aclarubicin and mitoxantrone. The mechanisms of anthracyclines include DNA intercalation (molecules insert between the two strands of DNA), generation of highly reactive free radicals that damage intercellular molecules and topoisomerase inhibition. Actinomycin is a complex molecule that intercalates DNA and prevents RNA synthesis. Bleomycin, a glycopeptide isolated from Streptomyces verticillus, also intercalates DNA, but produces free radicals that damage DNA. This occurs when bleomycin binds to a metal ion, becomes chemically reduced and reacts with oxygen. Mitomycin is a cytotoxic antibiotic with the ability to alkylate DNA.
Combination chemotherapy involves treating a patient with a number of different drugs simultaneously. The drugs differ in their mechanism and side effects. The biggest advantage is minimizing the chances of resistance developing to any one agent. Also, the drugs can often be used at lower doses, reducing toxicity. A prominent example is the combination of doxorubicin and cyclophosphamide (A/C).

"Resistance to chemotherapy" occurs when cancerous cells are not inhibited or killed by the treatment, at least at the concentration applied. In other words, the cancerous cells are resisting the effects of the chemotherapy. The term "sensitivity to chemotherapy" has a corresponding meaning.

The term "biomarker" or "marker" is widespread in the art and may broadly denote a biological molecule and/or a detectable portion thereof (e.g. a nucleic acid, a peptide or a lipid such as a glycolipid) whose qualitative and/or quantitative evaluation in an individual is predictive or informative (e.g., predictive, diagnostic and/or prognostic) with respect to one or more aspects of the individual's phenotype and/or genotype, such as, for example, with respect to the status of the individual. E.g. the biomarker is predictive or informative with respect to the outcome for chemotherapeutic treatment of a cancer in an individual. A biomarker is expressed ("expression of the biomarker") if the biomarker is detectable with methods known in the art. Therefore expression of biomarkers encompasses not only expression at nucleic acid level (DNA and/or RNA) and protein level but also expression (presence) of other biological structures on or in the cells such as glycolipids or the activity of a protein.
As used herein, the terms "patient" and "individual" are used interchangeably to refer to an animal. Preferentially, the individual is a mammal such as mouse, rat, cow, pig, goat, chicken dog, monkey or human. More preferentially, the individual is a human.

The term "tag" as used herein refers to the coupling of the antigen-binding fragment, e.g. an antibody or fragment thereof, to other molecules, e.g. particles, enzymes or enzyme fragments (for enzyme based detection of antibody binding), fluorophores, haptens like biotin, or larger surfaces such as culture dishes and microtiter plates. In some cases the coupling results in direct immobilization of the antigen-binding fragment, e.g. if the antigen-binding fragment is coupled to a larger surface of a culture dish. In other cases this coupling
results in indirect immobilization, e.g. an antigen-binding fragment coupled directly or indirectly (via e.g. biotin) to a magnetic bead is immobilized if said bead is retained in a magnetic field. In further cases the coupling of the antigen-binding fragment to other molecules does not result in a direct or indirect immobilization but allows for enrichment, separation, isolation, and detection of cells according to the present invention, e.g. if the antigen-binding fragment is coupled to a fluorophore which then allows discrimination of labeled cells and non-labeled cells, e.g. via flow cytometry methods, like FACSorting, or fluorescence microscopy.
The term "antigen-binding fragment" as used herein refers to any moiety that binds preferentially to the desired target molecule of the cell, i.e. the antigen.
The term "moiety" comprises, e.g., an antibody or antibody fragment.
The term "antibody" as used herein refers to polyclonal or monoclonal antibodies, which can be generated by methods well known to the person skilled in the art. The antibody may be of any species, e.g. murine, rat, sheep, human. For therapeutic purposes, if non-human antigen binding fragments are to be used, these can be humanized by any method known in the art. The antibodies may also be modified antibodies (e.g. oligomers, reduced, oxidized and labeled antibodies).
The term "antibody" comprises both intact molecules and antibody fragments, such as Fab, Fab , F(ab')2, Fv and single-chain antibodies. Additionally, the term "antigen-binding fragment" includes any moiety other than antibodies or antibody fragments that binds preferentially to the desired target molecule of the cell. Suitable moieties include, without limitation, oligonucleotides known as aptamers that bind to desired target molecules, carbohydrates, lectins or any other antigen binding protein (e.g. receptor-ligand interaction). The linkage (coupling) between antibody and tag or particle can be covalent or non-covalent. A covalent linkage can be, e.g. the linkage to carboxyl-groups on polystyrene beads, or to NH2 or SH2 groups on modified beads. A non-covalent linkage is e.g. via biotin-avidin or a fluorophore- coupled-particle linked to anti-fluorophoreantibody. Methods for coupling antibodies to particles, fluorophores, haptens like biotin or larger surfaces such as culture dishes are well known to the skilled person in the art.

### Embodiments

In one embodiment of the present disclosure a sample to be tested is achieved by a biopsy, e.g. a punch biopsy, a fine needle aspirate, or a surgical removal of the tumor or parts of the tumor. The sample is then processed by dissociation to a single cell suspension for measuring SSEA4 or ST3GAL2 expression or ST3GAL2 activity. As an alternative, the sample is fixed and may or may not be used to prepare sections, e.g. FFPE (formalin fixed paraffin embedded) sections, before measuring SSEA4 or ST3GAL2 expression. As an alternative the sample is directly lysed to isolate nucleic acids or protein from the sample for measuring SSEA4 or ST3GAL2 expression. In all cases the sample processing may or may not contain a step of cell separation, e.g. by flow cytometric of magnetic sorting. Cell separation or cell enrichment of cancerous cells may be performed by using antigen-binding fragments (antibodies) against tumor markers such as EPCAM, CD34, CD99, CD117, CA15-3, MUC16, MUC1, ErbB2/HER2, PSMA or MCSP.

In one embodiment of the present disclosure a sample to be tested is provided from a patient suffering from a cancer, i.e. human breast cancer. Determining the presence (expression) of SSEA4 in the test sample is performed by using an antigen-binding fragment such as a monoclonal antibody against SSEA4 which is labeled with a tag such as fluorophore. The presence (detection) of SSEA4 on cells of the sample to be tested, determined e.g. by flow cytometry or fluorescence microscopy, is indicative for a poor prognosis associated with resistance to chemotherapy, i.e. the breast cancer of the patient is resistant to chemotherapeutic treatment such as A/C.
In one embodiment the test sample indicates more than 50% of cancerous cells expressing SSEA4. Then the patient has a high risk that the tumor is resistant to chemotherapy.

In one embodiment of the present disclosure a sample to be tested is provided from a patient suffering from a cancer, i.e. RCC. Detecting the expression level (on protein level) of ST3GAL2 in the test sample is performed by using an antigen-binding fragment such as a monoclonal antibody against ST3GAL2 which is labeled with a tag such as a fluorophore. The cells of the sample are permeabilized and labeled with the anti-ST3GAL2 antibody. Cells from an individual not suffering from any cancer are used as reference cells. As an alternative, healthy tissue of the patient is used as a reference. These cells are also permeabilized and labeled with the anti-ST3GAL2 antibody. The expression level of ST3GAL2 in these reference cells are used as control level. An increase of the expression level (protein level) of ST3GAL2 in cells of the sample to be tested compared to the expression level (protein level) of ST3GAL2 of the reference cells (control level), determined e.g. by flow cytometry, is indicative for a poor prognosis, i.e. the RCC of the patient is resistant to chemotherapeutic treatment such as A/C.
In one embodiment the test sample indicates an increase of expression level (protein level) of ST3GAL2 of the cancerous cells of more than 50% compared to the control level (reference cells). Then the patient has a high risk that the tumor is resistant to chemotherapy.

In one embodiment of the present disclosure a sample to be tested is provided from a patient suffering from a cancer, i.e. ovarian cancer. Detecting the expression level (on nucleic acid level) of ST3GAL2 in the test sample is performed, e.g. by using Northern blot hybridization analysis, reverse-transcription-based PCR assays, RNA microarrays, or DNA microarrays. The cells of the sample are lysed and nucleic acids, e.g. mRNA or total RNA, are isolated. In some assays this isolation step may not be necessary, e.g. direct PCR on the lysed cells. Cells from an individual not suffering from any cancer are used as reference cells. As an alternative, healthy tissue of the patient is used as a reference and nucleic acids, e.g. mRNA or total RNA, are isolated. In some assays this isolation step may not be necessary, e.g. direct PCR on the lysed cells. The expression level of ST3GAL2 in these reference cells are used as control level. An increase of the expression level (nucleic acid level) of ST3GAL2 in cells of the sample to be tested compared to the expression level (nucleic acid level) of ST3GAL2 of the reference cells (control level), determined e.g. by PCR, is indicative for a poor prognosis, i.e. the ovarian cancer of the patient is resistant to chemotherapeutic treatment such as A/C.
In one embodiment the test sample indicates an increase of expression level (nucleic acid level) of ST3GAL2 of the cancerous cells of more than 50% compared to the control level (reference cells). Then the patient has a high risk that the tumor is resistant to chemotherapy.

In one embodiment of the present disclosure a sample to be tested is provided from a patient suffering from a cancer, i.e. breast cancer. Detecting the expression level (on lipid or protein level) of SSEA4 or ST3GAL2 in the test sample is performed, e.g. by using Western blot hybridization analysis, ELISAs, or protein microarrays. The cells of the sample are lysed and proteins are isolated. In some assays this isolation step may not be necessary, e.g. direct analysis of the lysed cells. Cells from an individual not suffering from any cancer are used as reference cells. As an alternative, healthy tissue of the patient is used as a reference and proteins are isolated. In some assays this isolation step may not be necessary, e.g. direct analysis of the lysed cells. The expression level of SSEA4 or ST3GAL2 in these reference cells are used as control level. An increase of the expression level of SSEA4 or ST3GAL2 in cells of the sample to be tested compared to the expression level of SSEA4 or ST3GAL2 of the reference cells (control level), determined e.g. by ELISA, is indicative for a poor prognosis, i.e. the breast cancer of the patient is resistant to chemotherapeutic treatment such as A/C.
In one embodiment the test sample indicates an increase of expression level of SSEA4 or ST3GAL2 of the cancerous cells of more than 50% compared to the control level (reference cells). Then the patient has a high risk that the tumor is resistant to chemotherapy.

In one embodiment of the present disclosure a sample to be tested is provided from a patient suffering from a cancer, i.e. breast cancer. Detecting the activity level of ST3GAL2 in the test sample is performed, e.g. by ELISAs or substrate turnover assays. The cells of the sample are lysed and proteins are isolated. In some assays this isolation step may not be necessary, e.g. direct analysis of the lysed cells. Cells from an individual not suffering from any cancer are used as reference cells. As an alternative, healthy tissue of the patient is used as a reference and proteins are isolated. In some assays this isolation step may not be necessary, e.g. direct analysis of the lysed cells. The activity level of ST3GAL2 in these reference cells are used as control level. An increase of the activity level of ST3GAL2 in cells of the sample to be tested compared to the activity level of ST3GAL2 of the reference cells (control level), determined e.g. by ELISA, is indicative for a poor prognosis, i.e. the breast cancer of the patient is resistant to chemotherapeutic treatment such as A/C.
In one embodiment the test sample indicates an increase of activity level of ST3GAL2 of the cancerous cells of more than 50% compared to the control level (reference cells). Then the patient has a high risk that the tumor is resistant to chemotherapy.

### Examples

The following examples are intended for a more detailed explanation of the invention but without restricting the invention to these examples.

### Example 1: The expression of 23 cell surface markers on patient derived breast cancer xenografts is affected by chemotherapeutic treatment

An antibody screening was performed to identify novel cell surface biomarkers for breast cancer subpopulations showing an increased resistance to chemotherapeutic treatment. The screening was based on a library of 45 antibodies directed against human surface proteins (Table 1).

These antibody candidates have been selected based on our experience to be expressed on subpopulations of tumor cells. Pieces of patient derived breast tumor tissue were grafted into a cohort of mice (50 to 100 animals per tumor model). These tumor models were shown to preserve the morphology, molecular characteristics, and drug response profile of the original patient tumors (Marangoni et al., Clin Cancer Res 13, 3989 - 3998 (2007)). Upon stable establishment of the tumors (pre-treatment stage), indicated by tumor volumes of 150-350 mm³, we started the treatment using a standard care doxorubicin/cyclophosphamide (A/C) combination. After tumor shrinking to volumes of 14 - 63mm³, the nodules were removed (residual tumors stage). At the same day, tumors of untreated mice were removed (untreated stage) to serve as direct controls. In addition, a group of animals with residual tumors were kept until the disease relapsed to volumes similar to the pre-treated stage (regrown stage, Figure 1). After removing the tumors at the specific time points, the tissue was gently dissociated to obtain a single cell suspension while preserving cell surface epitopes to exclude a protocol induced bias. Subsequently, the sample was stained for mouse specific markers to exclude cells of murine origin from the analysis as well as for the screening candidates and analyzed by multi-parameter flow cytometry (Figure 2). Tumors from four different breast cancer patients were analyzed independently. In total, the expression of 13 markers (2 to 9 per tumor model) was enriched and the expression of 10 markers (1 to 5 per tumor model) was decreased during chemotherapy (Figure 3). Interestingly, 87% (20 of 23) of these markers came back to their background expression level as observed in the untreated stage upon regression of the disease (regrown stage, Figure 3).

**Tab. 1: Antibodies used for the screening approach**

| **Antigen** | **Clone** | **Vendor** | **Titer** |
|---|---|---|---|
| AN2/MCSP | 1E6.4 | Miltenyi Biotec | **1:11** |
| ABCB5 | Polyclonal rabbit IgG | Bioss | **1:50** |
| CaSR | Polyclonal rabbit IgG | Enzo Life Science | **1:50** |
| CD9 | M-L13 | BD Biosciences | **1:11** |
| CD10 | 97C5 | Miltenyi Biotec | **1:11** |
| CD15/SSEA1 | VIMC6 | Miltenyi Biotec | **1:11** |
| CD20 | LT20 | Miltenyi Biotec | **1:11** |
| CD24 | 32D12 | Miltenyi Biotec | **1:11** |
| CD26 | FR 10-11G9 | Miltenyi Biotec | **1:11** |
| CD34 | AC136 | Miltenyi Biotec | **1:11** |
| CD38 | IB6 | Miltenyi Biotec | **1:11** |
| CD44 | DB105 | Miltenyi Biotec | **1:11** |
| CD49a | TS/27 | BioLegend | **1:20** |
| CD49b | Y418 | eBioscience | **1:20** |
| CD49c | ASC-1 | BioLegend | **1:20** |
| CD49d | MZ18-24A9 | Miltenyi Biotec | **1:11** |
| CD49e | NKI-SAM1 | Miltenyi Biotec | **1:11** |
| CD49f | GoH3 | Miltenyi Biotec | **1:11** |
| CD61 | Y2/51 | Miltenyi Biotec | **1:11** |
| CD66 (a,c,d,e) | TET2 | Miltenyi Biotec | **1:11** |
| CD71 | AC102 | Miltenyi Biotec | **1:11** |
| CD90 | DG3 | Miltenyi Biotec | **1:11** |
| CD105 (Endoglin) | 43A4E1 | Miltenyi Biotec | **1:11** |
| CD117 | AC126 | Miltenyi Biotec | **1:11** |
| CD122 | Tu27 | BioLegend | **1:11** |
| CD133/1 | W6B3C1 | Miltenyi Biotec | **1:11** |
| CD133/2 | 293C3 | Miltenyi Biotec | **1:11** |
| CD138 | B-B4 | Miltenyi Biotec | **1:11** |
| CD146 | 541-10B2 | Miltenyi Biotec | **1:11** |
| CD166 | 3A6 | BioLegend | **1:6** |
| CD271 (NGF Receptor) | ME20.4-1.H4 | Miltenyi Biotec | **1:11** |
| CD309 (KDR/VEGF-R2) | ES8-20E6 | Miltenyi Biotec | **1:11** |
| CD324 (Ecad) | 67A4 | Miltenyi Biotec | **1:11** |
| CD325 (Ncad) | 8C11 | eBioscience | **1:11** |
| CD326 (EpCAM) | HEA-125 | Miltenyi Biotec | **1:11** |
| CD338 (ABCG2) | 5D3 | BD Biosciences | **1:20** |
| CD340 (Her2/neu) | 24D2 | BioLegend | **1:20** |
| DRD5 | Polyclonal rabbit IgG | Bioss | **1:100** |
| Lgr5 DA03 | DA03 | Miltenyi Biotec | **1:11** |
| ROR1 | 2A2 | Miltenyi Biotec | **1:11** |
| Sca1 | D7 | Miltenyi Biotec | **1:11** |
| SSEA4 | REA101 | Miltenyi Biotec | **1:11** |
| TGFbetaR | Polyclonal goat IgG | BD Biosciences | **1:11** |
| TRA-1-60 | REA157 | Miltenyi Biotec | **1:11** |
| TRA-1-81 | REA246 | Miltenyi Biotec | **1:11** |

The percentage of cells expressing CD146 was reduced in three out of four tumor models indicating it as the most sensitive subpopulation targeted during A/C treatment. Strikingly, we identified a subpopulation of tumor cells expressing stage-specific embryonic antigen-4 (SSEA4) to be strongly enriched during chemotherapeutic treatment in all of the analyzed tumor models. Both CD44 and CD133, for which a correlation with a cancer stem cell and drug resistance phenotype already has been described, only showed enrichment in one out of the four models.

### Example 2: SSEA4 is a marker for cells resistant to chemotherapy treatment

To further investigate the significance of SSEA4 expression during breast cancer treatment, we repeated the analysis of this marker in three of the tumor models. In addition, we included the pre-treatment stage into the analysis to exclude any change that might be induced by the time point or overall size of the tumor. The number of SSEA4 positive cells was significantly (p < 0.001, n = 8) enriched upon A/C treatment in all analyzed tumors (Figure 4). In addition, in three out of four tumor models the fraction of positive cells was reduced to background levels after the treatment was stopped and the tumors relapsed. In tumor model HBCx-17 we observed a de novo reduced sensitivity for A/C treatment in 19 animals and one tumor that did not respond to the treatment at all. Interestingly, the amount of SSEA4 positive cells was increased in these more (42.2%) and fully resistant tumors (98.5%) compared to the sensitive tumors (36.8%) in the regrown stage (data not shown). To evaluate if the known de novo resistance of further tumor models also correlates with the frequency of the SSEA4 positive tumor cell population, we compared tumors from patients which are sensitive (n = 6) or resistant (n = 4) to A/C treatment. Strikingly, three out of the four resistant tumor models showed higher percentages of SSEA4 positive cells than all of the six sensitive tumors (Figure 5). In two of the resistant tumor models, almost all of the cells expressed SSEA4. To evaluate if this observation is limited to breast cancer or may be more general, we analyzed Clear cell renal cell carcinoma (RCC), an independent tumor entity shown to be de novo resistant to chemotherapy in more than 95% of patients (Cohen, Herbert T.; McGovern, Francis J. (2005), New England Journal of Medicine 353 (23): 2477-90). Primary RCC as well as healthy kidney tissue from the same patient was dissociated and analyzed for SSEA4 expression. Strikingly, in all of the analyzed patients (n = 3) the expression of SSEA4 was strongly increased in the tumor tissue with almost all tumor cells expressing this marker (Figure 6).

To investigate further whereas cells surviving chemotherapy upregulate SSEA4 during the treatment or if already positive cells show an increased de novo resistance, we treated cell lines showing variable fractions of SSEA4 positive cells with chemotherapeutic drugs in vitro. A primary tumor cell line derived from model HBCxl7 showed reproducible growth partly in an adherent and in a suspension phenotype. Interestingly, the cells growing in suspension showed a higher SSEA4 expression compared to the adherent cells (Figure 7). To evaluate the sensitivity of both subpopulations to chemotherapeutic treatment, we determined the IC50 values (n = 3) for eight commonly used drugs (Figure 7 and data not shown). For five out of these, Cisplatine, Mafosfamide, 5-Fluorouracil, doxorubicin, and Docetaxel, the suspension cells showed higher IC50 values, indicating an increased resistance to those drugs. One of the drugs, Etoposide, showed no difference, and for two molecules, Topotecan and Irinotecan, decreased IC50 values of the suspension cells were observed. Etoposide, Topotecan, and Irinotecan inhibit the enzymes topoisomerase I and II, thus causing double-strand DNA breaks. Therefore, SSEA4 positive cells seem to be more resistant for DNA synthesis and transcription inhibitors but equal or less resistant to topoisomerase inhibitors.

To directly examine the phenotype of cells surviving the treatment, another purely adherent cell line derived from model HBCx17 was treated with increasing concentrations of either Mafosfamide, 4-Hydroxycyclophosphamide, or Adriamycine® (n = 4). In every case, the surviving population showed a significantly higher fraction of SSEA4 positive cells (Figure 7).

### Example 3: SSEA4 expression indicates a mesenchymal phenotype

The results of the mRNA and the miRNA analysis strongly indicate that the expression of SSEA4 correlates with a more mesenchymal phenotype. As EMT was shown to be correlated with drug resistance (Ganapati V. Hegde et al., PLOS ONE 2012, 7(10):e45647), we wanted to examine if also the expression of SSEA4 is increased upon EMT induction. To induce a mesenchymal transition, the epithelial breast cell line MCF10A was treated with TGFβ1 at concentrations of 10 and 20 ng/ml. Upon treatment, almost all cells changed their morphology from a compacted colony type to an elongated fibroblastic shape (Figure 8). Previously known key EMT markers like EpCAM, E-cadherin, Vimentin, and Fibronectin were regulated as expected, proving the efficiency of EMT induction using our method (Figure 8 and data not shown). In addition, also the invasive potential of treated cells was strongly increased (data not shown). Strikingly, the fraction of SSEA4 positive cells was increased upon EMT induction verifying the correlation between SSEA4 expression and a more mesenchymal phenotype (Figure 8).

### Example 4: SSEA4and/or ST3GAL2 are a highly significant prognostic markers in breast cancer patients

Huge collections of gene expression data with matched clinical outcome of breast cancer patients are already available. However, due to the nature of the SSEA4 epitope, its expression cannot be directly monitored by transcriptome profiling. Therefore, we evaluated the prognostic value of its synthetizing enzyme ST3GAL2 in a large public clinical microarray database of breast tumors from 2,977 patients (Gyorffy B et al., Breast Cancer Res Treatment, 2010 Oct;123(3):725-31.). When using the whole dataset, no difference in the clinical outcome was observed whereas highly significant differences (p < 0.01) towards a poorer prognosis for patients expressing high levels of ST3GAL2 in the ER- patients, PR-patients, and double negative patients were found independent of the treatment (Figure 9). When focusing on patients treated with chemotherapy, a highly significant reduction of relapse free survival independent of the tumor subtype (p < 0.01, HR 1.91), in ER- patients (p < 0.01, HR 2.97), and in double negative patients (p < 0.01, HR 3.08) was observed (Figure 9). This strongly supports the importance of SSEA4 and ST3GAL2 as diagnostic markers to predict chemotherapy resistance and outcome in all major breast cancer subtypes. Not only when applying relapse free survival as an endpoint but also for distant metastasis-free survival, patients expressing high levels of ST3GAL2 had a worse prognosis, indicating a role of the SSEA4 positive subpopulation also during metastasis (Figure 9).

### Example 5: SSEA4/ST3GAL2 is a significant prognostic marker and indicates resistance to chemotherapy in other tumor entities

To evaluate if this observation is limited to breast cancer or may be more general, we also analyzed the prognostic value of ST3GAL2 in a large public clinical microarray database of ovarian tumors from 1,464 patients (Gyorffy B et al, Endocrine-Related Cancer. 2012 Apr 10;19(2):197-208.). Already when using the whole dataset, a significant difference towards a worse clinical outcome was observed with respect to progression free survival (p < 0.05) as well as post progression survival (p < 0.05, Figure 10). When focusing on patients treated with chemotherapy, again the level of significance was strongly increased for both endpoints (p < 0.01, Figure 10) indicating that the use of SSEA4 and ST3GAL2 as diagnostic markers may not be limited to breast cancer.

## Claims

1. The use of the biomarkers SSEA4 and/or ST3GAL2 for assessing the outcome for chemotherapeutic treatment of a cancer in an individual, wherein said cancer is selected from the group consisting of human breast cancer, human renal cell carcinoma (RCC) and human ovarian cancer if the biomarker is SSEA4 and wherein said cancer is human breast cancer if the biomarker is ST3GAL2 and wherein the chemotherapeutic drugs used in said chemotherapeutic treatment are DNA synthesis and transcription inhibitors.

2. A method for assessing the prognosis associated to resistance to chemotherapy in an individual having a cancer, the method comprising detecting expression of SSEA4 in a test sample from the individual Wherein the expression of SSEA4 in the test sample is indicative of a poor prognosis, and wherein said cancer is selected from the group consisting of human breast cancer, human renal cell carcinoma (RCC) and human ovarian cancer, and wherein the chemotherapeutic drugs used in said chemotherapy are DNA synthesis and transcription inhibitors.

3. The method of claim 2, wherein the expression of SSEA4 on more than 50% of the cancerous cells of said sample is indicative of a poor prognosis.

4. The method of claim 2 or 3, wherein the expression of SSEA4 is determined by staining with an antibody or fragment thereof.

5. A method for assessing the prognosis associated to resistance to chemotherapy in an individual having a cancer, the method comprising a) Detecting the expression or activity level of ST3GAL2 in a sample from the individual b) Comparing the detected expression or activity level to a control level c) Determining said prognosis based on the comparison of b) Wherein the control level is a good prognosis control level and an increase of the expression or activity level compared to the control level is determined as poor prognosis, and wherein said cancer is selected from the group consisting of human breast cancer and human ovarian cancer, and wherein the chemotherapeutic drugs used in said chemotherapy are DNA synthesis and transcription inhibitors.

6. The method of claim 5, wherein said increase is at least 50% compared to said control level.

7. The method of claim 5 or 6 wherein said expression of the ST3Gal2 is determined on mRNA level or protein level.

8. The method of claim 5 or 6 wherein said activity of the ST3Gal2 protein is determined on protein level.

## Patentansprüche

1. Verwendung der Biomarker SSEA4 und/oder ST3GAL2 zur Beurteilung des Ergebnisses einer chemotherapeutischen Behandlung eines Krebses in einer Person, wobei der Krebs ausgewählt ist aus der Gruppe bestehend aus menschlichem Brustkrebs, menschlichem Nierenzellkarzinom (RCC) und menschlichem Ovarialkarzinom, wenn der Biomarker SSEA4 ist, und wobei der Krebs menschlicher Brustkrebs ist, wenn der Biomarker ST3GAL2 ist, und wobei die in der chemotherapeutischen Behandlung verwendeten chemotherapeutischen Arzneimittel DNA-Synthese- und Transkriptionshemmer sind.

2. Verfahren zur Beurteilung der Prognose in Verbindung mit der Resistenz gegen Chemotherapie bei einer Person mit einem Krebs, wobei das Verfahren den Nachweis der Expression von SSEA4 in einer Testprobe aus der Person umfasst,
wobei die Expression von SSEA4 in der Testprobe auf eine schlechte Prognose hinweist, und wobei der Krebs ausgewählt ist aus der Gruppe bestehend aus menschlichem Brustkrebs, menschlichem Nierenzellkarzinom (RCC) und menschlichem Ovarialkarzinom, und wobei die in der Chemotherapie verwendeten chemotherapeutischen Arzneimittel DNA-Synthese- und Transkriptionshemmer sind.

3. Verfahren nach Anspruch 2, wobei die Expression von SSEA4 auf mehr als 50 % der Krebszellen der Probe eine schlechte Prognose anzeigt.

4. Verfahren nach Anspruch 2 oder 3, wobei die Expression von SSEA4 durch Färbung mit einem Antikörper oder einem Fragment davon bestimmt wird.

5. Verfahren zur Beurteilung der Prognose in Verbindung mit der Resistenz gegen eine Chemotherapie bei einer Person mit einem Krebs, wobei das Verfahren Folgendes umfasst:
a) Nachweisen des Expressions- oder Aktivitätsniveaus von ST3GAL2 in einer Probe der Person,
b) Vergleichen des erfassten Expressions- oder Aktivitätsniveaus mit einem Kontrollniveau,
c) Bestimmen der Prognose basierend auf dem Vergleich von b),
wobei das Kontrollniveau ein gutes Prognosekontrollniveau ist und ein Anstieg des Expressions- oder Aktivitätsniveaus im Vergleich zum Kontrollniveau als schlechte Prognose definiert wird, und wobei der Krebs ausgewählt ist aus der Gruppe bestehend aus menschlichem Brustkrebs und menschlichem Ovarialkarzinom, und wobei die in der Chemotherapie verwendeten chemotherapeutischen Arzneimittel DNA-Synthese- und Transkriptionshemmer sind.

6. Verfahren nach Anspruch 5, wobei der Anstieg mindestens 50 % gegenüber dem Kontrollniveau beträgt.

7. Verfahren nach Anspruch 5 oder 6, wobei die Expression des ST3Gal2 auf mRNA- oder Proteinebene bestimmt wird.

8. Verfahren nach Anspruch 5 oder 6, wobei die Aktivität des ST3Gal2-Proteins auf Proteinebene bestimmt wird.

## Revendications

1. Utilisation des biomarqueurs SSEA4 et/ou ST3GAL2 pour évaluer le résultat d'un traitement chimiothérapeutique d'un cancer chez un individu, ledit cancer étant choisi dans le groupe constitué par le cancer du sein humain, un carcinome à cellules rénales (CCR) humain et le cancer de l'ovaire humain si le biomarqueur est SSEA4 et ledit cancer étant le cancer du sein humain si le biomarqueur est ST3GAL2 et lesdits médicaments chimiothérapeutiques utilisés dans ledit traitement chimiothérapeutique étant des inhibiteurs de transcription et de synthèse d'ADN.

2. Procédé d'évaluation du pronostic associé à une résistance à une chimiothérapie chez un individu atteint d'un cancer, le procédé comprenant la détection de l'expression de SSEA4 dans un échantillon test provenant de l'individu, ladite expression de SSEA4 dans l'échantillon test indiquant un mauvais pronostic, et ledit cancer étant choisi dans le groupe constitué par le cancer du sein humain, un carcinome à cellules rénales (CCR) humain et le cancer de l'ovaire humain, et lesdits médicaments chimiothérapeutiques utilisés dans ladite chimiothérapie étant des inhibiteurs de transcription et de synthèse d'ADN.

3. Procédé selon la revendication 2, ladite expression de SSEA4 dans plus de 50 % des cellules cancéreuses dudit échantillon indiquant un mauvais pronostic.

4. Procédé selon la revendication 2 ou 3, ladite expression de SSEA4 étant déterminée par coloration avec un anticorps ou un fragment de celui-ci.

5. Procédé d'évaluation du pronostic associé à une résistance à une chimiothérapie chez un individu atteint d'un cancer, le procédé comprenant a) la détection du niveau d'expression ou d'activité de ST3GAL2 dans un échantillon provenant d'un individu, b) la comparaison du niveau d'expression ou d'activité détecté à un niveau témoin, c) la détermination dudit pronostic sur la base de la comparaison de b), ledit niveau témoin représentant un bon niveau de contrôle de pronostic et une augmentation du niveau d'expression ou d'activité par rapport au niveau témoin étant déterminée comme étant un mauvais pronostic, et ledit cancer étant choisi dans le groupe constitué par le cancer du sein humain et le cancer de l'ovaire humain, et lesdits médicaments chimiothérapeutiques utilisés dans ladite chimiothérapie étant des inhibiteurs de transcription et de synthèse d'ADN.

6. Procédé selon la revendication 5, ladite augmentation étant supérieure ou égale à 50 % par rapport audit niveau témoin.

7. Procédé selon la revendication 5 ou 6, ladite expression de la protéine ST3Gal2 étant déterminée par le taux d'ARNm ou le taux de protéine.

8. Procédé selon la revendication 5 ou 6, ladite activité de la protéine ST3Gal2 étant déterminée par le taux de protéine.
